# EUROPEAN PATENT APPLICATION

(11) **EP 2 404 588 A1**
(43) Date of publication of application: **11.01.2012**
(21) Application number: 10748710.0
(22) Date of filing: 01.03.2010
(51) Int. Cl.: A61J 1/14, A61J 1/03, A61K 9/20, A61K 31/27

(54) **PACKAGE OF SOLID PHARMACEUTICAL PREPARATION**

(30) Priority: 02.03.2009 JP 2009048665
(71) Applicant: Astellas Pharma Inc., Tokyo 103-8411 (JP)
(72) Inventor: YOSHITA, Tomohiro, Tokyo 103-8411 (JP); IZUMI, Fumi, Tokyo 103-8411 (JP); OHI, Hiroshi, Tokyo 103-8411 (JP)
(74) Representative: Grünecker, Kinkeldey, Stockmair & Schwanhäusser
(86) International application number: PCT/JP2010/053247
(87) International publication number: WO 2010/101115

(57) **Abstract**

Provided is a package of a solid pharmaceutical preparation capable of preventing an unpleasant odor and coloring due to hydrolysis progressing during storage of a solid pharmaceutical preparation containing 1-{[(α-isobutanoyloxyethoxy)carbonyl]aminomethyl}-1-cyclohe xane acetic acid.

A package of a solid pharmaceutical preparation housing a solid pharmaceutical preparation containing 1-{[(α-isobutanoyloxyethoxy)carbonyl]aminomethyl}-1-cyclohe xane acetic acid includes a PTP package, an adsorbent, and an outer packaging material. The PTP package includes the solid pharmaceutical preparation, a container sheet for housing the solid pharmaceutical preparation, and a cover sheet having gas permeability sealing the solid pharmaceutical preparation. The adsorbent includes at least zeolite. The outer packaging material includes a gastight material to house and seal the adsorbent and the PTP package.

## Description

### Technical Field

The present invention relates to a package of a solid pharmaceutical preparation containing 1-{[(α-isobutanoyloxyethoxy)carbonyl]aminomethyl}-1-cyclohe xane acetic acid.

### Background Art

1-{[(α-isobutanoyloxyethoxy)carbonyl]aminomethyl}-1-c yclohexane acetic acid is a prodrug of gabapentin, which is a γ-aminobutyric acid (GABA) derivative, has a high bioavailability as gabapentin when administered either orally or directly into the colon of a mammal (Patent Document 1, Non-patent Document 1 and Non-patent Document 2), and a sustained release oral drug is known as a pharmaceutical preparation thereof (e.g., refer to Patent Document 3).

This 1-{[(α-isobutanoyloxyethoxy)carbonyl]aminomethyl}-1-cyclohe xane acetic acid is hydrolyzed under the presence of water, whereby related substances, acetaldehyde, and carbon dioxide are generated. Therefore, while storing a solid pharmaceutical preparation containing 1-{[(α-isobutanoyloxyethoxy)carbonyl]aminomethyl}-1-cyclohe xane acetic acid, this hydrolyzation progresses by way of the moisture in the storage environment or moisture contained in the solid pharmaceutical preparation ; and generation of an unpleasant odor and coloring occur. In the case of soft packing such as a bag, a malfunction may occur such as the packing material swelling due to the gas evolved by hydrolysis.

Therefore, in order to prevent these malfunctions, it has been considered that the solid pharmaceutical preparation containing 1-{[(α-isobutanoyloxyethoxy)carbonyl]aminomethyl}-1-cyclohe xane acetic acid be provided in a state where it is filled in a plastic bottle or the like along with a well-known silica gel or the like as a drying agent. According to this configuration, the moisture in the bottle decreases by a drying agent such as silica gel and the progression of hydrolysis is delayed, whereby generation of an unpleasant odor and progression of coloring are delayed.

However, in the case of this solid pharmaceutical preparation being filled in a plastic bottle or the like, the progression of hydrolysis can be delayed by the drying agent until the unsealing of the bottle; however, there has been the possibility that the solid pharmaceutical preparation contacts with moisture in the air when the bottle is unsealed for dividing at a pharmacy or the like, and then hydrolysis progresses, whereby coloring and an unpleasant odor occur after dividing.
In addition, in experiments placing 28 tablets of 665 mg of a solid pharmaceutical preparation containing 1-{[(α-isobutanoyloxyethoxy)carbonyl]aminomethyl}-1-cyclohe xane acetic acid in an aluminum bag along with 9 g of silica gel and storing it for 1 week at 50°C, it was observed that coloring and an unpleasant odor occurred. Therefore, development of a means capable of effectively preventing generation of an unpleasant odor and coloring due to hydrolysis during storage of the solid pharmaceutical preparation has been demanded.

On the other hand, a technique for enclosing a package in which a solid pharmaceutical preparation containing a compound that tends to undergo alteration under a low pH environment is PTP packaged or the like in a bag formed by aluminum together with zeolite in order to suppress this alteration of this compound without using a strongly basic substance (Patent Document 3), and the fact that synthetic zeolite as well as natural zeolite can be used as a drying agent in a package of a solid pharmaceutical preparation made by PTP packaged capsules and the drying agent being enclosed and wrapping gastight in a film (Patent Document 4) have been disclosed.

Patent Document 1: PCT International Patent Publication No.WO02/100347
Patent Document 2: Published Japanese Translation of PCT Application No.2008-518971 (paragraphs 0003 and 0004)
Patent Document 3: Japanese Patent Application Publication No.2003-137778 (paragraphs 0004 to 0006, 0008, and 0020 to 0026)
Patent Document 4: Japanese Patent No. 3906485 (paragraphs 0010 and 0046)

Non-patent Document 1: J. Pharmacol. Exp. Ther. 2004, 311: 315-323
Non-patent Document 2: J. Pharmacol. Exp. Ther. 2004, 311: 324-333

### Disclosure of the InventionProblems to be Solved by the Invention

The present invention has been made taking the above-mentioned situation into account, and an object thereof is to provide a package of a solid pharmaceutical preparation using PTP packaging that is capable of preventing generation of an unpleasant odor and coloring due to hydrolysis progressing during storage of a solid pharmaceutical preparation containing 1-{[(α-isobutanoyloxyethoxy)carbonyl]aminomethyl}-1-cyclohe xane acetic acid, and that can be handled with ease at a pharmacy or the like.
In addition, another object of the present invention is to provide a package of a solid pharmaceutical preparation for suppressing progression of hydrolysis and stably maintaining the solid pharmaceutical preparation by removing new moisture generated by hydrolysis.

It should be noted that Patent Document 3 discloses that alteration of compounds that tend to undergo alteration under a low pH environment can be prevented using zeolite, and Patent Document 4 merely discloses that the relative humidity inside a package of a solid pharmaceutical preparation sealed gastight can be effectively regulated using zeolite. Therefore, technique is not known that solves the characteristic problems of solid pharmaceutical preparation containing 1-{[(α-isobutanoyloxyethoxy)carbonyl]aminomethyl}-1-cyclohe xane acetic acid in that generation of an unpleasant odor and coloring due to hydrolysis progressing during storage.

### Means for Solving the Problems

According to a package of a solid pharmaceutical preparation related to Claim 1, the problems are solved by a package housing a solid pharmaceutical preparation containing 1-{[(α-isobutanoyloxyethoxy)carbonyl]aminomethyl}-1-cyclohe xane acetic acid, in which the package includes a PTP package, an adsorbent, and an outer packaging material; the PTP package contains the solid pharmaceutical preparation, a container sheet in which a housing portion housing the solid pharmaceutical preparation is formed, and a cover sheet having gas permeability sealing the solid pharmaceutical preparation; the adsorbent having at least zeolite; and the outer packaging material is formed by a gastight material and houses and seals the adsorbent and the PTP package. Herein, it should be noted that, another embodiment of sealing includes gastight sealing.

In addition, the PTP package is not particularly limited so long as the gas permeability via the cover sheet is ensured; however, it is preferable when the cover sheet side is arranged inside of the outer packaging material so as to face the outer packaging material side, and the adsorbent is configured so as to be arranged between the cover sheet and the outer packaging material facing each other.

Moreover, the adsorbent is not particularly limited so as long as being arranged so as to be able to absorb moisture inside the outer packaging material, gas, odor, and moisture contained in the solid pharmaceutical preparation; however, it is preferable when it is arranged so as to contact the cover sheet.

Furthermore, the effective pore size of the zeolite is preferably at least 4Å.

In addition, the zeolite is preferably 0.09 to 50 g relative to 28 tablets containing 300 mg of 1-{[(α-isobutanoyloxyethoxy)carbonyl]aminomethyl}-1-cyclohe xane acetic acid.
Furthermore, the particle size of the zeolite is preferably 0.9 to 5 mm.

The zeolite is preferably 1 to 600 wt% relative to the amount of 1-{[(α-isobutanoyloxyethoxy)carbonyl]aminomethyl}-1-cyclohe xane acetic acid.

In addition, the cover sheet is not particularly limited so long as the gas permeability is ensured; however, it is preferable when it is configured so as to be composed of a sheet in which air holes are formed in a material selected from the group consisting of aluminum, polyvinyl chloride, polypropylene, and polyvinylidene chloride, or in another embodiment, glassine paper.

Furthermore, it is preferable when the adsorbent is made by the zeolite in powder or grain form being housed in a container having gas permeability.

### Effects of the Invention

According to the invention of Claim 1, by a solid pharmaceutical preparation containing 1-{[(α-isobutanoyloxyethoxy)carbonyl] aminomethyl}-1-cyclohexane acetic acid being housed and sealed along with an adsorbent formed by zeolite in the outer packaging material formed by a gastight material, moisture in the outer packaging material and moisture contained in the solid pharmaceutical preparation are adsorbed in the adsorbent; and it becomes possible to suppress the hydrolysis reaction between moisture in the outer packaging material and in the solid pharmaceutical preparation with the 1-{[(α-isobutanoyloxyethoxy)carbonyl]aminomethyl}-1-cyclohe xane acetic acid, while at the same time it becomes possible to adsorb aldehyde and carbon dioxide evolved from this hydrolysis reaction. Thus, generation of an unpleasant odor and coloring during storage of the solid pharmaceutical preparation containing 1-{[(α-isobutanoyloxyethoxy)carbonyl]amznomethyl}-1-cyclohe xane acetic acid can be prevented.

In addition, since the cover sheet has gas permeability, in a state where the adsorbent is arranged outside the PTP package, the solid pharmaceutical package containing 1-{[(α-isobutanoyloxyethoxy)carbonyl]aminomethyl}-1-cyclohe xane acetic acid can be PTP packaged. It is possible to provide a PTP package that does not require dividing at a pharmacy or the like while generation of an unpleasant odor and coloring during storage of this solid pharmaceutical preparation are prevented.
Since zeolite that lacks deliquescent property but has high adsorbability is used as the adsorbent, moisture and the like adsorbed to the adsorbent will not affect the solid pharmaceutical preparation in the PTP package even if the adsorbent and the cover sheet are contacting, as in Claim 3.
Although carbon dioxide which has a molecular weight of 44 and aldehyde cannot be adequately adsorbed when the effective pore size of the zeolite is less than 4Å, according to Claim 4, since it is configured in a manner that the effective pore size of the zeolite is greater than or equal to 4Å, adsorption of water, carbon dioxide, and acetaldehyde can be achieved.
According to Claims 5 and 7, it is possible to adequately adsorb water, aldehyde, and carbon dioxide by way of an adsorbent having zeolite.
According to Claim 6, fast adsorption under low relative humidity conditions can be achieved.
According to Claim 8, the solid pharmaceutical preparation containing 1-{[(α-isobutanoyloxyethoxy)carbonyl]aminomethyl}-1-cyclohe xane acetic acid can be PTP packaged in a state where the adsorbent is arranged outside of the PTP package; and it is possible to provide a PTP package that does not require dividing at a pharmacy or the like while generation of an unpleasant odor and coloring during storage of this solid pharmaceutical preparation are prevented.
According to Claim 9, since zeolite in powder or grain form is used, fast adsorption under low relative humidity conditions can be achieved.

### Brief Description of the Drawing

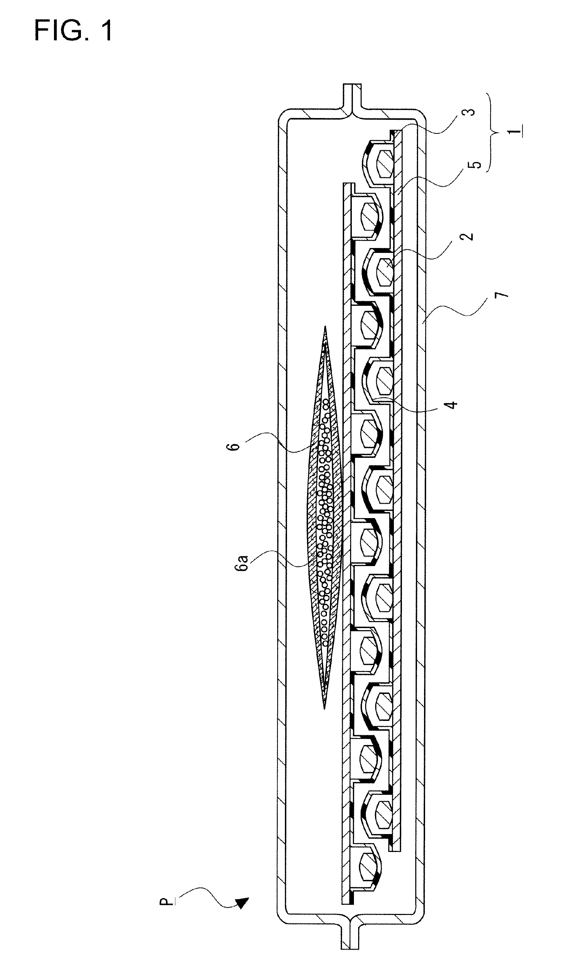

FIG. 1 is a schematic cross-sectional explanatory view showing a package of a solid pharmaceutical preparation according to an embodiment of the present invention.

### Explanation of Reference Numerals

- P: Package of solid pharmaceutical preparation
- 1: PTP package
- 2: Solid pharmaceutical preparation
- 3: Container sheet
- 4: Housing portion
- 5: Cover sheet
- 6: Adsorbent
- 6a: Zeolite
- 7: Outer packaging material

### Best Modes for Carrying out the Invention

In the present specification, "stable" indicates being stable to heat, temperature and moisture, for example. This is established as the related substances of 1-{[(α-isobutanoyloxyethoxy)carbonyl] aminomethyl}-1-cyclohexane acetic acid after 6 months at 40°C and 75% relative humidity being no more than 3 wt% of the total amount of 1-{[(α-isobutanoyloxyethoxy)carbonyl]aminomethyl}-1-cyclohe xane acetic acid, for example.
In the present specification, "coloring" indicates exhibiting color other than white relative to white in external appearance. For example, when the color difference ΔE between the solid pharmaceutical preparation immediately after production and the solid pharmaceutical preparation after being maintained at 40°C and 75% relative humidity is measured with a colorimeter, it is defined as having coloration when ΔE > 3.

The package of the solid pharmaceutical preparation P of the present embodiment is a package in which solid pharmaceutical preparations 2 containing 1-{[(α-isobutanoyloxyethoxy)carbonyl]aminomethyl}-1-cyclohe xane acetic acid that have been PTP packaged are enclosed in an outer packaging material 7 of soft wrapping formed by gastight material together with an adsorbent 6 formed by zeolite 6a.
The package of the solid pharmaceutical preparation P includes a PTP package 1, the adsorbent 6 containing the zeolite 6a, and the outer packaging material 7 formed by a gastight material housing the adsorbent 6 and the PTP package 1, as shown in FIG. 1.

The PTP package 1 is composed of a container sheet 3 in which an housing portions 4 that house the solid pharmaceutical preparation are formed, and a cover sheet 5 that covers the housing portions 4. The solid pharmaceutical preparations 2 containing 1-{[(α-isobutanoyloxyethoxy)carbonyl]aminomethyl}-1-cyclohe xane acetic acid are housed in the housing portions 4.
The container sheet 3 is not particularly limited so long as a sheet can wrap the solid pharmaceutical preparation, and may have gas permeability. Examples of the container sheet 3 includes a well-known plastic sheet such as a polyvinyl chloride (PVC) sheet, a polypropylene (PP) sheet, a polyvinylidene chloride (PVDC) sheet, and a polychlorotrifluoroethylene sheet; well-known aluminum sheets; and the like. A plurality of housing portions 4 of concave shape housing the solid pharmaceutical preparations are provided in the container sheet 3, as shown in FIG. 1.

The cover sheet 5 may be a sheet composed of a material having moisture permeability, for example, and in other embodiments, may be well-known plastic sheets such as a polyvinyl chloride (PVC) sheet, polypropylene (PP) sheet, polyvinylidene chloride (PVDC) sheet and a polychlorotrifluoroethylene sheet, a well-known aluminum sheet that has fine air holes, or the like. In a further embodiment, glassine paper may be used.

The solid pharmaceutical preparation 2 is a sustained-release oral drug composed of a tablet, and contains 1-{[(α-isobutanoyloxyethoxy)carbonyl]aminomethyl}-1-cyclohe xane acetic acid, which is represented by the chemical Formula (1), as the active component. 1-{[(α-isobutanoyloxyethoxy)carbonyl]aminomethyl}-1-cyclohe xane acetic acid is a prodrug of gabapentin (Formula (2)), which is a γ-aminobutyric acid (GABA) derivative.

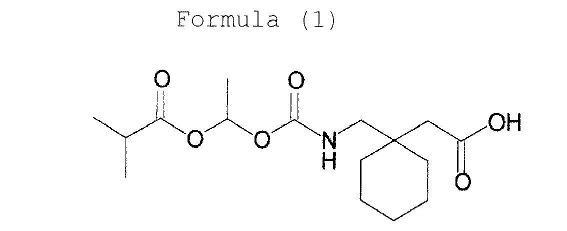

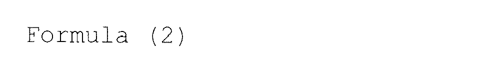

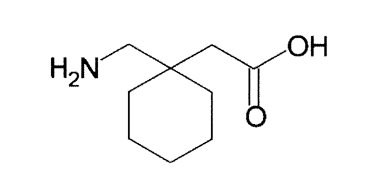

Continuous-release oral drugs of 1-{[(α-isobutanoyloxyethoxy)carbonyl]aminomethyl}-1-cyclohe xane acetic acid can be administered to a patient suffering either a disease or disorder for which the gabapentin having a medicinal action is known to be effective in therapy, or those discovered in the future. The symptoms for which gabapentin is prescribed, and the symptoms for which drugs containing gabapentin is effective are epilepsy, depression, anxiety, psychosis, dementia, schizophrenia, syncopal attacks, hypokinesis, craniopathy, neurogenerative disorder, panic attacks, pain {particularly, neuropathic pain (e.g., posttherpetic neuralgia), myalgia, and skeletal pain}, restless leg syndrome, hot flashes, enuresis, inflammatory lesion (i.e. arthrosis), insomnia, gastrointestinal injury, alcohol/cocaine addiction, ethanol withdrawal syndrome, vulval lesion, prospermia, glutamatergic, and the like.

This drug can be administered to a patient even as a precaution against the above-mentioned diseases or disorders. For that reason, this drug can be administered as a precaution to patients that have a propensity for epilepsy, depression, anxiety, psychosis, syncopal attacks, hypokinesis, craniopathy, neurogenerative disorder, panic attacks, pain (particularly, neuropathic pain, myalgia, and skeletal pain), inflammatory lesion (i.e. arthrosis), insomnia, gastrointestinal injury, ethanol withdrawal syndrome, prospermia, and vulval lesion.

Therefore, this drug can be used in order to prevent a certain disease or disorder and in order to treat another disease or disorder at the same time (e.g., prevention of psychosis, treatment of gastrointestinal injury, prevention of neuropathic pain, treatment of ethanol withdrawal syndrome and the like). During the early stages of a viral infection, this drug can be used in combination with another medicine such as an antiviral medicine, and can inhibit or reduce the events resulting from the neuropathic disorder.

The preferred dose range for oral administration of gabapentin is usually approximately 100 mg/day to approximately 3600 mg/day, and 1-{[(α-isobutanoyloxyethoxy)carbonyl]aminomethyl}-1-cyclohe xane acetic acid or alternatively a pharmacologically permitted salt or pharmacologically permitted solvate thereof can be adjusted so as to provide an equimolar amount of gabapentin. The dose range can be easily determined according to a method known to those skilled in the art. In certain embodiments, the amount of 1-{[(α-isobutanoyloxyethoxy)carbonyl]aminomethyl}-1-cyclohe xane acetic acid in a solid pharmaceutical preparation is in the range of approximately 50 mg to approximately 800 mg, in certain embodiments it becomes approximately 100 mg to approximately 800 mg, and in certain embodiments, it is approximately 300 mg to approximately 700 mg. In addition, as a dosage regimen, oral administration is done 1 to 3 times per day. It should be noted that the applied dosage is expected to be suitably determined depending on the individual case, taking symptoms, age, gender and the like into consideration.

1-{[(α-isobutanoyloxyethoxy)carbonyl]aminomethyl}-1-c yclohexane acetic acid is hydrolyzed under the presence of water, whereby 1 mol of acetaldehyde and carbon dioxide are respectively yielded from 1 mol of water and 1-{[(α-isobutanoyloxyethoxy)carbonyl]aminomethyl}-1-cyclohe xane acetic acid, respectively, as shown by the following reaction formulas (3) and (4).

1-{[(α-isobutanoyloxyethoxy)carbonyl] aminomethyl}-1-cyclohexane acetic acid + H₂O -> gabapentin + isobutyl alcohol + CH₃CHO + CO₂

Gabapentin -> gabapentin lactam + H₂O

In the case of the solid pharmaceutical preparations 2 being sealed inside of the outer packaging material 7, so long as water exists in the outer packaging material 7, the 1-{[(α-isobutanoyloxyethoxy)carbonyl]aminomethyl}-1-cyclohe xane acetic acid is degraded by way of the reactions of reaction formulas (3) and (4), whereby acetaldehyde and carbon dioxide evolve. In this case, since the same amount of water as the 1 mol of water used in the reaction of reaction formula (3) is generated by the reaction of reaction formula (4), the water generated by reaction formula (4) causes a new hydrolytic reaction to progress.
Other than the 1-{[(α-isobutanoyloxyethoxy)carbonyl]aminomethyl}-1-cyclohe xane acetic acid that is the active component, the solid pharmaceutical preparation 2 contains a release-rate controlling polymer, an excipient, a diluent, a glidant, a lubricant, a thickening inhibitor, a surfactant, a buffer solution, a dye, a humectant, an emulsifier, a pH buffer, a stabilizer, a thickener, a disintegrant, and a coloring agent as additives. The additives can be appropriately added in the proper amounts as one type or a combination of two or more types.

The release-rate controlling polymer is a glyceryl ester such as glyceryl monostearate, glyceryl behenate, glyceryl palmito-stearate, lauroyl macrogol glyceride, stearoyl macrogol glyceride or any combination of the above. In certain embodiments, the release-rate modifying polymer is glyceryl behenate. Another fat and/or wax release-rate modifying polymer includes lauryl alcohol, myristyl alcohol, stearyl alcohol, cetyl alcohol, cetostearyl alcohol, palmitoyl alcohol, ouricury wax, hydrogenated vegetable oil, Candelilla wax, esparto wax, stearic acid, paraffin wax, beeswax, glycowax, castor wax, and carnauba wax.

The excipient includes starch, sugar, gelatin, malt, rice, wheat flour, chalk, silica gel, sodium stearate, glycerol monostearate, talc, sodium chloride, glycerol, propylene glycol, water, ethanol, and the like.
The diluent can be added in order to set an applicable size for compressing the drug, and can increase the volume thereof. Examples of useful diluents include dibasic calcium phosphate, dibasic calcium phosphate dehydrate, calcium sulfate, dicalcium phosphate, tricalcium phosphate, lactose, cellulose including microcrystalline cellulose, kaolin, mannitol, sodium chloride, dry starch, pregelatinized starch, compressible sugar, mannitol, and any combinations of the above. In certain embodiments, a single diluent is selected from dibasic calcium phosphate and microcrystalline cellulose. In embodiments in which the diluent is dibasic calcium phosphate, the drug can include an amount of the diluent that is in the range of approximately 30 wt% to approximately 50 wt%, and in certain embodiments, in the range of approximately 35 wt% to approximately 45 wt%. In embodiments in which the diluent is microcrystalline cellulose, the drug can include an amount of diluent that is in the range of approximately 5 wt% to approximately 20 wt%, and in certain embodiments, in the range of approximately 10 wt% to approximately 16 wt%.

The glidant is contained in the drug of the present invention, and lowers the sticking effect during production, film formation, and/or drying. Examples of useful glidants are talc, magnesium stearate, glycerol monostearate, colloidal silicon dioxide, precipitated silicon dioxide, and combinations of any of the above. In certain embodiments, the glidant is colloidal silicon dioxide. The drug can contain less than approximately 2 wt% glidant, and in certain embodiments, less than approximately 1 wt% glidant.

Lubricants and anti-adherents can be included in the drug of the present invention in order to aid in processing. Examples of useful lubricants and/or anti-adherents are calcium stearate, glyceryl behenate, glyceryl monostearate, magnesium stearate, mineral oil, polyethylene glycol, sodium stearyl fumarate, sodium lauryl sulfate, sodium dodecyl sulfate, stearic acid, talc, hydrogenated vegetable oil, zinc stearate, and combinations of any of the above. In certain embodiments, the lubricant is glyceryl monostearate. In certain embodiments, the lubricant is magnesium stearate. The drug can include the lubricant and/or anti-adherent in a range of approximately 1 wt% to approximately 13 wt%, and in certain embodiments, in a range of approximately 4 wt% to approximately 10 wt%.

Examples of useful surfactants in the drug of the present invention include pharmaceutically acceptable anionic surfactants, cationic surfactants, amphoteric (amphiphatic/amphiphilic) surfactants, non-ionic surfactants, polyethyleneglycol esters or ethers, and combinations of any of the above. Preferred pharmaceutically acceptable anionic surfactants include monovalent alkyl carboxylates, acyl lactylates, alkyl ether carboxylates, N-acyl sarcosinates, polyvalent alkyl carbonates, N-acyl glutamates, fatty acid-polypeptide condensates, sulfuric acid esters, alkyl sulfates such as sodium lauryl sulfate and sodium dodecyl sulfate, ethoxylated alkyl sulfates, ester linked sulfonates such as docusate sodium and dioctyl sodium succinate, alpha olefin sulfonates, or phosphated ethoxylated alcohols.
The solid pharmaceutical preparation 2 of the present embodiment is composed of a molded tablet; however, it may be an encapsulated formulation.

The adsorbent 6 has zeolite 6a housed in a bag composed of a gas permeable material. In the present embodiment, spherical synthetic zeolite having an effective pore size of 4Å and particle size of 4 to 20 meshes (approximately 0.9 to 5 mm) is used; however, it is not limited thereto, and natural zeolite or artificial zeolite may be used. Zeolite of sheet form may be used as the adsorbent 6.
Natural zeolite contains SiO₂, Al₂O₃, alkali metal, alkali earth metal as chemical components in addition to H₂O, is the general term for a group of minerals including a dimensional mesh structure, and is also called boiling stone. All have a plurality of pores of molecular level size in the surface, and thereby have excellent adsorbent action. Furthermore, they have an ion-exchange property. As a result, toxic substances such as heavy metals and radioactive materials can be adsorbed. Furthermore, they have a water retention function of adsorbing and retaining moisture. In addition, in the case of being placed in an alkaline environment, they can promote neutralization thereof.
Additionally, the size can be set to a suitable size by pulverizing from the produced state.
Artificial zeolites are made by chemically treating coal ash in which silica and alumina account for approximately 80% at high temperature and high pressure along with caustic soda and the like.

Synthetic zeolite and artificial zeolite are zeolites in which materials having properties resembling natural zeolite are formed by an industrial method from a variety of materials. As a result, the adsorptive property, ion-exchange property, etc. are improved. In addition, natural zeolite differs in being produced as grains having regular shapes such as spheres and columns; therefore, time and effort for pulverizing and the like is unnecessary.

In the present embodiment, so long as being a zeolite that can maintain the stability of the solid pharmaceutical preparation containing the 1-{[(α-isobutanoyloxyethoxy)carbonyl]aminomethyl}-1-cyclohe xane acetic acid, it is not limited; however, zeolite having an effective pore size of at least 4Å can be exemplified, for example.
The effective pore size is limited to at least 4Å because, although moisture in the outer packaging material 7 can be adsorbed when the effective pore size is less than 4Å, since carbon dioxide and acetaldehyde generated according to reaction formula (3) cannot be adsorbed thereby, a residual amount of carbon dioxide and acetaldehyde generated under the presence of moisture remaining in small amounts in the outer packaging material 7 cannot be removed.

Although the amount of zeolite 6a used is not particularly limited so long as being an amount that can maintain the stability of the solid pharmaceutical preparation containing the 1-{[(α-isobutanoyloxyethoxy)carbonyl]aminomethyl}-1-cyclohe xane acetic acid, in the case of housing 28 tablets of the solid pharmaceutical preparation 2 containing 300 mg of 1-{[(α-isobutanoyloxyethoxy)carbonyl]aminomethyl}-1-cyclohe xane acetic acid per 1 tablet inside the outer packaging material 7 having a volume of 200 mL, 0.09 to 50 g of the zeolite 6a is used, in another embodiment, 0.09 to 15 g thereof is used, in yet another embodiment, 0.09 to 3 g thereof is used, and in yet another further embodiment, 0.09 to 2 g thereof is used. In addition, relative to an amount of 100 mg of 1-{[(α-isobutanoyloxyethoxy)carbonyl]aminomethyl}-1-cyclohe xane acetic acid, 0.001 to 0.6 g of the zeolite 6a is used, in another embodiment, 0.001 to 0.20 g thereof is used, in yet another embodiment, 0.001 to 0.05 g thereof is used, and in yet another further embodiment, 0.001 to 0.02 g thereof is used, for example. Furthermore, as the proportion of zeolite to the amount of 1-{[(α-isobutanoyloxyethoxy)carbonyl]aminomethyl}-1-cyclohe xane acetic acid, for example, 1 to 600 wt% is used, in another embodiment, 1 to 200 wt% is used, in yet another embodiment, 1 to 40 wt% is used, and in yet another further embodiment, 1 to 25 wt% is used.
The moisture contained in the solid pharmaceutical preparation 2 is approximately 0.01 to 1 wt% of the pharmaceutical preparation, for example.
A bag composed of a non-woven fabric is used as the container composed of a breathable material housing the zeolite 6a, for example.

The outer packaging material 7 housing the adsorbent 6 and the PTP package 1 is formed by a sealable film material having a property of being able to prevent efflux and influx of gas such as water vapor. As the film constituting the outer packaging material 7, for example, high density polyethylene films, poly vinylidene chloride films, high density polyethylene laminated paper, poly vinylidene chloride laminated paper, polychlorotrifluoroethylene films, polypropylene films and the like, as well as films on which a barrier layer has been laminated on the film can be exemplified, and in another embodiment, it is an aluminum foil.
Pillow packaging is preferable as the form of the bag of the outer packaging material 7. In addition, on a pillow packaged side, a known plastic fastener that seals, after an end side has been unsealed by tearing, the opening thereof to be freely sealed or unsealed may be formed.

Although the volume of the outer packaging material is not particularly limited so long as the PTP packages and adsorbent can be arranged and sealed therein, it can be exemplified as 10 to 1000 mL, and in another embodiment, as 10 to 300 mL, for example.

The PTP packages 1 and the adsorbent 6 are arranged inside of the outer packaging material 7 as shown in FIG. 1.
In the PTP packages 1 of the present embodiment, 7 tablets of the solid pharmaceutical preparation 2 are arranged in two rows, with a total of 14 tablets being housed therein. As shown in FIG. 1, two sheets of the PTP packages 1 are housed in the outer packaging material 7 so that a side of the cover sheet 5 faces the outer packaging material 7 by setting the side of the container sheet 3 on the inside. The two sheets of PTP packages 1 are arranged so as to alternate such that the housing 4 of one of the PTP packages 1 faces the concaved surface of other housing portions 4 on the other PTP package 1, and are configured such that the stacked thickness of the two sheets of PTP packages 1 is a minimum. It thereby becomes possible to reduce the volume of the outer packaging material 7, and decrease the amount of water contained in the outer packaging material 7.

As shown in FIG. 1, the adsorbent 6 is arranged between the cover sheet 5 of one of the PTP packages 1 and the outer packaging material 7 so as to contact with the cover sheet 5. The adsorbent 6 assumes the role of a filter that covers the cover sheet 5, and the adsorbent 6 is present at a location near the cover sheet 5, and thus is able to promptly remove moisture contained by the solid pharmaceutical preparations 2.
The adsorbent 6 may be arranged so that the odor and moisture inside the outer packaging material can be adsorbed, and a total of two of the adsorbents 6 may be arranged not only between one of the cover sheets 5 and the outer packaging material 7, but also at two sites between the cover sheet 5 and the outer packaging material 7.

### Examples

Although the present invention will be explained more specifically hereinafter by way of the Examples, the present invention is not to be limited thereto.

### (Pharmaceutical Preparation Example)

The solid pharmaceutical preparations 2 composed of a 655 mg tablet containing 1-{[(α-isobutanoyloxyethoxy)carbonyl]aminomethyl}-1-cyclohe xane acetic acid (300 mg) were prepared according to a method described in Published Japanese Translation of PCT Application No. 2008-518971.

A tablet with a total weight of 655.0 mg was obtained by mixing 1-{[(α-isobutanoyloxyethoxy)carbonyl]aminomethyl}-1-cyclohe xane acetic acid (active component), calcium hydrogenphosphate (diluent), glycerin fatty acid ester (release-rate controlling polymer), talc (glident), light anhydrous silicic acid (glident), sodium lauryl sulfate (surfactant), and magnesium stearate (lubricant), and then compressing.
The composition per one tablet was 300 mg of 1-{[(α-isobutanoyloxyethoxy)carbonyl]aminomethyl}-1-cyclohe xane acetic acid, 259.1 mg of calcium hydrogenphosphate, 30.05 mg of glycerin fatty acid ester, 40.0 mg of talc, 2.7 mg of light anhydrous silicic acid, 12.0 mg of sodium lauryl sulfate, and 11.15 mg of magnesium stearate.

### (Examples 1 to 5)

28 tablets of the solid pharmaceutical preparation 2 obtained by the above-mentioned pharmaceutical preparation example were PTP packaged using the container sheet 3 composed of PVC and the cover sheet 5 composed of glassine paper (Oji Specialty Paper Co., Ltd.) to obtain the PTP package 1. The adsorbent 6 in which 1 g of spherical synthetic zeolite (Zeorum A-4 made by Tosoh Corporation) having an effective pore size of 4Å and particle size of approximately 0.9 to 5 mm placed in a bag composed of a non-woven fabric, and the PTP package 1 were wrapped gastight with aluminum pillow bag with a volume of 200 mL under conditions of 22°C and 55% relative humidity to obtain the package of solid pharmaceutical preparation P of Example 1.

Except for setting, the amount of synthetic zeolite to 3 g, the package of solid pharmaceutical preparation P of Example 2 was obtained by a similar procedure to Example 1.
In addition, except for setting the amount of the solid pharmaceutical preparation 2 to 30 tablets and the amount of synthetic zeolite to 2 g and an aluminum pillow bag with a plastic fastener in placed of the aluminum pillow bag, the package of solid pharmaceutical preparation of Examples 3 was obtained by a similar procedure to Example 1.
Furthermore, except for using 1.5 g of zeolite in sheet form (PET coat, Shinagawa Chemicals, Co. Ltd.) as the adsorbent 6 in place of the adsorbent in which 1 g of synthetic zeolite (Zeorum A-4 made by Tosoh Corporation) placed in a bag composed of a non-woven fabric, the package of solid pharmaceutical preparation P of Example 4 was obtained by a similar procedure to Example 1. In addition, except for making 30 tablets of the solid pharmaceutical preparation 2, the package of the solid pharmaceutical preparation P of Example 5 was obtained by a similar procedure to Example 1.

### (Comparative Examples 1 to 3)

14 tablets of the solid pharmaceutical preparation 2 obtained in the above-mentioned pharmaceutical preparation example were PTP packaged using the container sheet 3 composed of PVC and the cover sheet 5 composed of glassine paper to obtain the PTP package 1 of Comparative Example 1.
30 tablets of the solid pharmaceutical preparation 2 obtained in the above-mentioned pharmaceutical preparation example were PTP packaged using the container sheet 3 composed of PVC and the cover sheet 5 composed of glassine paper to obtain the PTP package 1. The adsorbent 6 in which 2 g of spherical synthetic zeolite (Zeorum A-4 made by Tosoh Corporation) having an effective pore size of 4Å and particle size of approximately 0.9 to 5 mm placed in a bag composed of a non-woven fabric, and the PTP package 1 were placed in an aluminum pillow bag with a volume of 200 mL under conditions of 22°C and 55% relative humidity, and then left in an opened state to obtain the package of solid pharmaceutical preparation P of Comparative Example 2.

28 tablets of the solid pharmaceutical preparation 2 obtained in the above-mentioned pharmaceutical preparation example were PTP packaged using the container sheet 3 composed of PP and the cover sheet 5 composed of aluminum to obtain the PTP package 1. The adsorbent 6 in which 5g of spherical synthetic zeolite (Zeorum A-4 made by Tosoh Corporation) having an effective pore size of 4Å and particle size of approximately 0.9 to 5 mm placed in a bag composed of a non-woven fabric, and the PTP package 1 were wrapped gastight with aluminum pillow bag with a volume of 200 mL under conditions of 22°C and 55% relative humidity to obtain the package of solid pharmaceutical preparation P of Comparative Example 3.

Each of the following experiments was performed with the packages of the solid pharmaceutical preparation P or the PTP packages 1 obtained in Examples 1 to 5 and Comparative Examples 1 to 3 as samples.

### (Test Example 1)

Each sample of Examples 1 to 5 and Comparative Examples 1 to 3 was defined as an unstored sample, and each sample thereof stored under conditions of 40°C and 75% relative humidity was defined as a stored sample. Each of the solid pharmaceutical preparations 2 packaged in a package was extracted, the color difference ΔE between the unstored samples and stored samples were measured using a colorimeter (CM-3500d, made by Konica Minolta) or the external appearance thereof was evaluated visually. In the coloring evaluation, examples with a color difference Δ > 3 were evaluated as being colored.

### (Test Example 2)

After each sample of Examples 1 to 4 and Comparative Examples 1 to 3 had been stored for 3 months (Examples 1, 2 and 4), 5 weeks (Example 3), or 1 week (Examples 1 to 3) under conditions of 40°C and 75% relative humidity, odor evaluation to evaluate the odor thereof immediately after unsealing the PTP package 1 was performed by sensory tests by means of healthy individuals. In the odor evaluation, "yes" defines a case of an odor being sensed and "none" defines a case of not being sensed.

### (Test Example 3)

After each sample of Examples 1 to 5 and Comparative Examples 1 to 3 had been stored for 6 months under conditions of 40°C and 75% relative humidity, each of the solid pharmaceutical preparations 2 packaged in the packages were extracted, and the total amount of related substances (IBA, gabapentin lactam) in the total amount of the solid pharmaceutical preparation 2 was quantified by HPLC method, and stability experiments were performed thereon. A reference value of the total amount of related substances in the total amount of the solid pharmaceutical preparation 2 was established at 3 wt%, and samples exceeding 3 wt% were determined as being outside the reference range and having inferior stability.

### (Results)

The results of Test Examples 1 to 3 are shown in Table 1.

| | | Example 1 | Example 2 | Example 3 | Example 4 | Example 5 | Comparative Example 1 | Comparative Example 2 | Comparative Example 3 |
|---|---|---|---|---|---|---|---|---|---|
| Test Example 1 | Coloring ΔE | 0.55 | 0.54 | 0.89 | 0.92 | Not measured | 5.46, 8.88 | 4.32, 11.14 | Not measured |
| | | | | | | White | | | (Greyish brown |
| | (storage period) | (3 months) | (3 months) | (5 weeks) | (3 months) | (6 months) | (3 days, 1 week) | (1 day, 1 week) | (6 months) |
| Test Example 2 | Odor | None | None | None | None | Not measured | Yes | Yes | Yes |
| | | | | | | | | | |
| | (storage period) | (3 months) | (3 months) | (5 weeks) | (3 months) | | (1 week) | (1 week) | (6 months) |
| Test Examples 3 | Stability (total amount of related substances/total amount of solid pharmaceutical preparation (wt%)) | 2.2 | Not measured | Not measured | Not measures | 1.78 | Not measured | Not measured | 10.53 |
| | (storage period) | (6 months) | | | | (6 months) | | | (6 months) |

With regard to the results of Test Example 1, although Examples 1 to 5 had not colored even after 5 weeks or 3 months storage, Comparative Examples 1 and 2 had colored after 1 week storage, as shown in the results of Table 1. From these results, it was found that, with samples not wrapped gastight with aluminum pillow bag, an unacceptable amount of coloring occurs; however, by wrapping the PTP packages 1 of Examples 1 to 5 gastight with aluminum pillow bag along with the adsorbent 6 containing the zeolite 6a, coloring could be effectively prevented.
With regards to the results of Test Example 2, there was no odor immediately after unsealing of the PTP package 1; however, for Comparative Examples 1 to 3, odor was evolved immediately after unsealing of the PTP packages 1. From these results, it was found that the generation of odor is effectively prevented by the packages of solid pharmaceutical preparation P of Examples 1 to 4.
With regards to the results of Test Example 3, the proportion of the total amount of related substances in the total amount of drug displaying stability of the solid pharmaceutical preparation 2 was within the reference range at 2.2 wt% for Example 1, and was within the reference range at 1. 78 wt% for Example 5, and thus had adequate stability; however, for Comparative Example 3, it was 10.53 wt%, which exceeds the reference range, and thus the required stability could not be obtained thereby. From these results, it was found that, in the case of configuring the PTP package 1 with only materials not having gas permeability, adequate stability of the active component was not obtained, and hydrolysis progressed to an extent that was unacceptable; however, by configuring the cover sheet 5 of the PTP package 1 with glassine paper, the stability of the active component could be improved, and the progression of hydrolysis could be effectively suppressed.

In addition, it was found that all of the criteria for the aspects of the coloring after 3 months storage, odor after 3 months storage, and stability of the active component of the solid pharmaceutical preparation 2 after 6 months storage were satisfied by the package 1 of the solid pharmaceutical preparation of Example 1.

## Claims

1. A package of a solid pharmaceutical preparation containing 1-{[(α-isobutanoyloxyethoxy)carbonyl]aminomethyl}-1-cyclohe xane acetic acid, the package comprising:
a PTP package;
an adsorbent; and
an outer packaging material,
the PTP package including the solid pharmaceutical preparation, a container sheet with a housing portion that houses the solid pharmaceutical preparation, and a cover sheet having gas permeability sealing the solid pharmaceutical preparation,
the adsorbent including at least zeolite,
the outer packaging material comprising a gastight material, and
the outer packaging material housing and sealing the adsorbent and the PTP package.

2. The package of the solid pharmaceutical preparation according to claim 1, wherein
the PTP package is disposed in the outer packaging material in a manner that a cover sheet side of the PTP package faces toward the outer packaging material, and
the adsorbent is disposed between the cover sheet and the outer packaging material that are facing each other.

3. The package of the solid pharmaceutical preparation according to claim 1, wherein the adsorbent is disposed so as to contact the cover sheet.

4. The package of the solid pharmaceutical preparation according to claim 1, wherein the zeolite has an effective pore size of at least 4Å.

5. The package of the solid pharmaceutical preparation according to claim 1, wherein the zeolite is 0.09 to 50 g for 28 tablets containing 300 mg of the 1-{[(α-isobutanoyloxyethoxy)carbonyl]aminomethyl}-1-cyclohe xane acetic acid.

6. The package of the solid pharmaceutical preparation according to claim 1, wherein the zeolite has a particle size of 0.9 to 5 mm.

7. The package of the solid pharmaceutical preparation according to claim 1, wherein the zeolite is 1 to 600 wt% by mass relative to amount of the 1-{[(α-isobutanoyloxyethoxy)carbonyl]aminomethyl}-1-cyclohe xane acetic acid.

8. The package of the solid pharmaceutical preparation according to claim 1, wherein the cover sheet comprises glassine paper or a sheet with air holes comprising a material selected from the group including aluminum, polyvinyl chloride, polypropylene, and polyvinylidene chloride.

9. The package of the solid pharmaceutical preparation according to claim 1, wherein the adsorbent comprises the zeolite in powdery or granular form contained within a container having gas permeability.
